# EUROPÄISCHE PATENTSCHRIFT

(11) **EP 0 967 926 B1**
(45) Veröffentlichungstag und Bekanntmachung des Hinweises auf die Patenterteilung: **10.09.2003**
(21) Anmeldenummer: 97903192.9
(22) Anmeldetag: 28.02.1997
(51) Int. Cl.: A61B 17/80, A61B 17/68

(54) **IMPLANTAT FÜR DIE OSTEOSYNTHESE**
OSTEOSYNTHESIS IMPLANT
IMPLANT POUR OSTEOSYNTHESE

(43) Veröffentlichungstag der Anmeldung: 05.01.2000
(73) Patentinhaber: SYNTHES AG Chur, 7002 Chur (CH)
(72) Erfinder: HERZOG, Daniel, CH-4460 Gelterkinden (CH); KAPP, Bernhard, D-79650 Schopfheim (DE)
(74) Vertreter: Lusuardi, Werther Giovanni, Dr.
(86) Internationale Anmeldenummer: CH9700074
(87) Internationale Veröffentlichungsnummer: WO98037825

(56) Entgegenhaltungen:
- WO-A-94/23654
- WO-A-95/26164

## Beschreibung

Die Erfindung betrifft ein Implantat für die Osteosynthese gemäss dem Oberbegriff des Patentanspruchs 1.

Aus dem Stand der Technik sind derartige Implantate für die Osteosynthese bereits bekannt und zwar einerseits solche aus Memory-Alloys (sogenannte "Gedächtnis-Legierungen") und anderseits solche aus gewöhnlichen Metalllegierungen jedoch mit elastischer Vorspannung. Die Nachteile dieser bekannten Implantate bestehen darin, dass beide Typen ("memory-alloys" und "non-memory-alloys") nicht bioresorbierbar sind und daher potentiell eine Zweitoperation zur Metallentfernung notwendig ist. Zusätzlich ist die Biokompatibilität bei den Memory-Alloys durch deren hohen Nickelanteil umstritten. Aufgrund der Tatsache, dass das metallische Implantat später entfernt werden muss, können dessen Fortsätze auch nicht mit einer Retentionsstruktur versehen werden. Ein weiterer Nachteil der bekannten Implantate besteht schliesslich darin, dass sie nur zwei Fortsätze aufweisen.

Aus der WO-A-9526164 ist ein Implantat für die Osteosynthese gemäss dem Oberbegriff des Anspruchs 1 bekannt. Nachteilig bei diesem bekannten Implantat ist aber die erhebliche und konstante Dicke des plattenförmigen Grundkörpers.

Hier will die Erfindung Abhilfe schaffen. Der Erfindung liegt die Aufgabe zugrunde, ein Implantat für die Osteosynthese zu schaffen, insbesondere für den maxillofazialen Bereich, mit dem sich die zu fixierenden Knochenteile einfach und sicher verbinden lassen und welches nach erfolgter Osteosynthese nicht mehr explantiert werden muss.

Die Erfindung löst die gestellte Aufgabe mit einem Implantat, welches die Merkmale des Anspruchs 1 aufweist.

Mit der Erfindung ist der Vorteil erzielbar, dass mit einem einzigen Implantat, welches mehrere Fortsätze aufweist, ein Mehrfragmentbruch (z.B. im Schädelbereich) mit nur einem Bauelement versorgt werden kann. Im Falle eines einfachen Bruchs (mit nur einer Frakturlinie) kann durch ein einziges Implantat mit mehreren Fortsätzen die Stabilität der Frakturversorgung erheblich erhöht werden. Ein weiterer Vorteil besteht schliesslich in der wesentlich verkürzten Operationszeit gegenüber bekannten Implantaten.

Eine bevorzugte Weiterbildung besteht darin, dass die Fortsätze des Implantats nicht senkrecht zur Grundkörper stehen, sondern gegeneinander konvergieren, so dass eine Vorspannung dieser Fortsätze resultiert. Die erzeugte Vorspannung bewirkt nun eine minimale Kompression auf die Fraktur und verkürzt damit die Dauer der Regeneration des Knochens. Der Winkel beträgt vorzugsweise maximal 20°.

Um das erfindungsgemässe Implantat optimal anwenden zu können ist eine spaltfreie Reposition der Knochenfragmente wichtig. Wenn diese gewährleistet ist, erfolgt das Vorbohren der Löcher für die in den Knochen einzubringenden Fortsätze des Implantats mittels eines Mehrfachbohrkopfes, der über den Frakturverlauf gelegt wird, so dass in einem einzigen Arbeitsgang sämtliche benötigten Bohrungen angebracht werden können. Danach wird das Implantat mittels eines geeigneten Instrumentes aufgenommen, welches eine allfällig vorhandene Konvergenz der Fortsätze neutralisiert (d.h. die Fortsätze werden mittels des Instrumentes parallel ausgerichtet), so dass das Implantat nun problemlos in die parallel ausgerichteten "Mehrfachbohrungen" eingedrückt und fixiert werden kann. Danach kann eine eventuell angebrachte Retentionshilfe entfernt werden.

Die durch die gegeneinander konvergierenden Fortsätze erzeugte Vorspannung bewirkt eine minimale Kompression auf die Fraktur und kann damit die Regeneration des Knochens begünstigen. Darüber hinaus sorgt die Vorspannung zusammmen mit der Retentionsstruktur der Fortsätze für einen besseren Halt des Implantates im Knochen.

Bei einer weiteren bevorzugten Ausführungsform sind die Fortsätze des Implantats als Hohlkörper mit einem zentralen Kanal ausgebildet, in welche ein Spreizkörper einführbar ist, um eine noch bessere Fixation zu erzielen.

Neben der Hauptanwendung für die Überbrückung von Frakturspalten, kann das erfindungsgemässe Implantat auch als Fixationskomponente für resorbierbare Folien, Membranen oder resorbierbare Osteosyntheseplatten verwendet werden. Die Folien, bzw. Membranen werden hauptsächlich dazu benutzt um
- Knochendefekte zu überbrücken;
- Orbitaböden zu rekonstruieren; und
- gezielt einen Knochenaufbau im Zahnbereich zu erreichen.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine perspektivische Ansicht eines erfindungsgemässen Implantats mit zwei Fortsätzen;
Fig. 2 einen Längsschnitt durch das Implantat nach Fig. 1;
Fig. 3 eine perspektivische Ansicht eines erfindungsgemässen Implantats mit drei Fortsätzen;
Fig. 4 eine perspektivische Ansicht eines erfindungsgemässen Implantats mit vier Fortsätzen;
Fig. 5 einen Längsschnitt durch den Fortsatz eines erfindungsgemässen Implantates mit einem Spreizkörper;
Fig. 6 eine Seitenansicht eines modifizierten Fortsatzes mit Widerhaken;
Fig. 7 eine Seitenansicht einer weiteren Variante eines Fortsatzes mit Kegelstumpfabschnitten;
Fig. 8 eine Seitenansicht einer weiteren Variante eines Fortsatzes mit Sägezähnen;
Fig. 9 einen Längsschnitt durch eine weitere Variante eines Fortsatzes mit nach oben gerichteten Sägezähnen;
Fig. 10 eine Seitenansicht einer weiteren Variante eines längsgeschlitzten Fortsatzes; und
Fig. 11 eine perspektivische Ansicht eines erfindungsgemässen Implantats mit zentrale Kanäle aufweisenden Fortsätzen, sowie einem dazu passenden Verschlussteil.

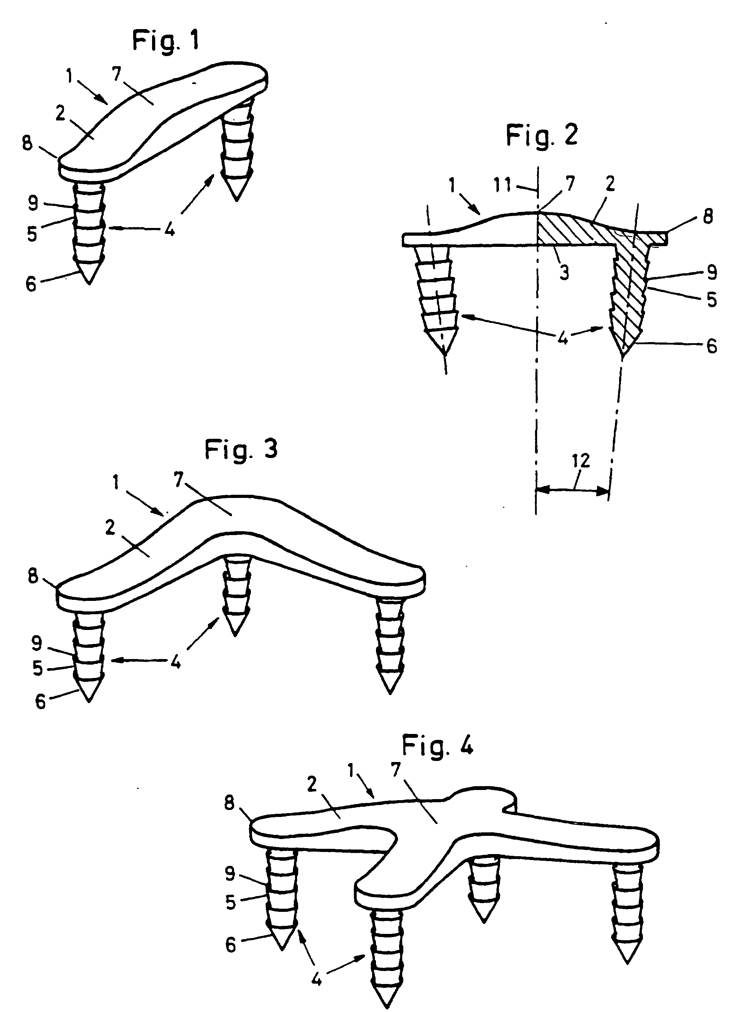

Das in den Fig. 1 und 2 dargestellte erfindungsgemässe Implantat besteht im wesentlichen aus einem plattenförmigen Grundkörper 1, der eine gewölbte Oberseite 2 und eine für den Knochenkontakt bestimmte, planare Unterseite 3 umfasst.

Der plattenförmige, z.B. rechteckförmige Grundkörper 1 kann entweder voll oder als Gitter ausgebildet sein, wobei die Dicke des plattenförmigen Grundkörpers 1 vom Zentrum 7 zur Peripherie 8 hin kontinuierlich abnimmt.

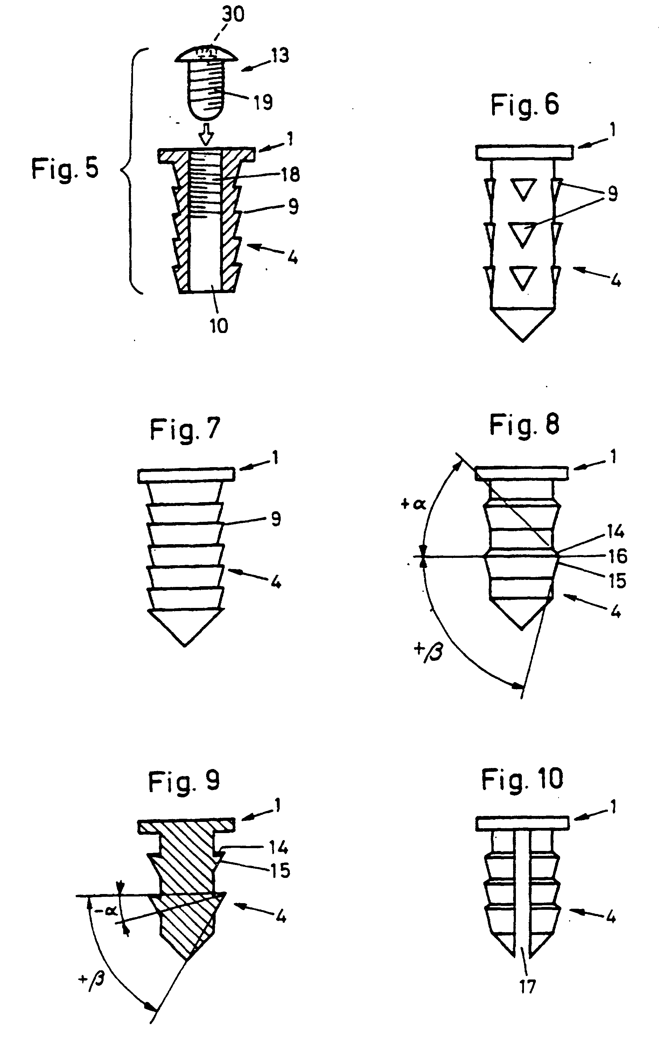

Die Unterseite 3 des Implantats weist zwei Fortsätze 4 auf, welche für die Implantation im Knochen bestimmt sind und deren Aussenflächen 5 mit einer Retentions-Struktur 9 in Form von sich zur Unterseite 3 hin konisch verbreiternden Kegelstumpfabschnitten versehen ist (Fig. 5 und 7).

Die kreiszylindrisch ausgebildeten Fortsätze 4 konvergieren gegeneinander und weisen gegenüber der Senkrechten 11 auf dem Grundkörper 1 einen Winkel 12 von 1° - 15°, vorzugsweise von 2° - 10° auf. Die Fortsätze 4 sind an ihrem freien Ende spitz ausgebildet.

Das gesamte Implantat besteht aus einem bioresorbierbaren Material, welches folgende Eigenschaften aufweist:
- Beibehaltung der Ausgangsstabilität über einen Zeitraum von 6 bis 8 Wochen;
- Plastische Verformbarkeit unter Wäremeinwirkung; und
- Aufquellbarkeit des Implantats im implantierten Zustand.

In Fig. 3 ist eine weitere Ausführungsform des erfindungsgemässen Implantats in Form einer Winkelplatte dargestellt mit drei Fortsätzen 4. Bei dieser Ausführungsform stehen die kreiszylindrisch ausgebildeten Fortsätze 4 im wesentlichen senkrecht zum Grundkörper 1 und sind an ihrem freien Ende 6 abgerundet.

In Fig. 4 ist eine weitere Ausführungsform des erfindungsgemässen Implantats in Form eines Sterns dargestellt mit vier Fortsätzen 4 an jedem der freien Sternzacken. Auch bei dieser Ausführungsform stehen die kreiszylindrisch ausgebildeten Fortsätze 4 im wesentlichen senkrecht zum Grundkörper 1 und sind an ihrem freien Ende 6 abgerundet.

Wie in Fig. 5 dargestellt kann der Fortsatz 4 als Hohlkörper mit einem zentralen Kanal 10 ausgebildet sein, in welchen ein - vorzugsweise bioresorbierbarer - Spreizkörper 13 einführbar ist, um den Fortsatz 4 mit seiner Retentionsstruktur 9 im Knochen zu verspreizen. Der Spreizkörper 13 ist vorzugsweise als Schraube mit dem Aussengewinde 19 ausgebildet, welche in das Innengewinde 18 des zentralen Kanals 10 einschraubbar ist. Zu diesem Zweck ist der Kopf des Spreizkörpers 13 mit einem Innensechskant 30 versehen, in welchen ein entsprechendes Eindrehinstrument eingeführt werden kann.

Wie in Fig. 6 dargestellt kann die Retentions-Struktur 9 bei allen Ausführungsformen auch aus gegen die Unterseite 3 gerichteten Widerhaken bestehen.

Die Fig. 8 - 10 zeigen Varianten der Fortsätze 4, welche als Retentionsstruktur Sägezähne 16 aufweisen.

Bei der in Fig. 8 gezeigten Variante weist der Winkel α, den die gegen den Grundkörper 1 gerichtete Fläche 14 der Sägezähne 16 mit der Ebene des Grundkörpers 1 einschliesst, einen positiven Wert auf, währenddem bei der in Fig. 9 dargestellten Variante der Winkel α einen negativen Wert aufweist.
In beiden Fällen ist der Absolutwert des Winkels α kleiner oder höchstens gleich gross wie der Winkel +β, den die gegen das freie Ende 6 gerichtete Fläche 15 der Sägezähne 16 mit der Ebene des Grundkörpers 1 einschliesst.

Fig. 10 zeigt eine Variante der Fortsätze 4 mit einem Längsschlitz 17 um die Verspreizung im Knochen zu erleichtern. Die Längsschlitze 17 können - wie in Fig. 10 gezeigt - nach unten offen ausgebildet sein, oder auch nach unten geschlossen sein.

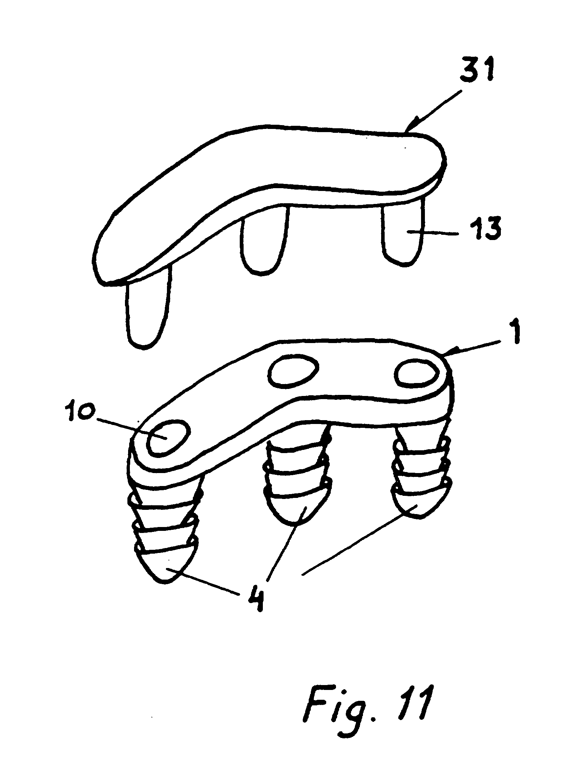

Fig. 11 zeigt schliesslich ein Implantat 1, welches drei einen zentralen Kanal 10 aufweisende Fortsätze 4 besitzt. Die zentralen Kanäle 10 können mittels des Verschlussteils 31, das drei untereinander verbundene Spreizkörper 13 aufweist, gleichzeitig verschlossen werden.

## Patentansprüche

1. Implantat für die Osteosynthese mit einem plattenförmigen Grundkörper (1), der eine Oberseite (2) und eine für den Knochenkontakt bestimmten Unterseite (3) umfasst, wobei
A) die Unterseite (3) des Implantats mindestens zwei Fortsätze (4) aufweist, welche für die Implantation im Knochen bestimmt sind;
B) die Aussenfläche (5) der Fortsätze (4) mit einer Retentions-Struktur (9) versehen ist; und
C) das gesamte Implantat aus einem bioresorbierbaren Material besteht, **dadurch gekennzeichnet, dass**
D) die Dicke des plattenförmigen Grundkörpers (1) vom Zentrum (7) zur Peripherie (8) hin kontinuierlich abnimmt.

2. Implantat nach Anspruch 1, **dadurch gekennzeichnet, dass** die Fortsätze (4) im wesentlichen senkrecht zum Grundkörper (1) angeordnet sind.

3. Implantat nach Anspruch 1, **dadurch gekennzeichnet, dass** die Fortsätze (4) gegeneinander konvergieren und gegenüber der Senkrechten (11) auf dem Grundkörper (1) vorzugsweise einen Winkel (12) von maximal 20° einschliessen.

4. Implantat nach Anspruch 3, **dadurch gekennzeichnet, dass** die Fortsätze (4) gegenüber der Senkrechten (11) auf dem Grundkörper (1) einen Winkel (12) von 1° - 15°, vorzugsweise von 2° - 10° einschliessen.

5. Implantat nach einem der Ansprüche 1 bis 4, **dadurch gekennzeichnet**, - dass die Fortsätze (4) im wesentlichen kreiszylindrisch ausgebildet sind und an ihrem freien Ende (6) vorzugsweise zugespitzt sind.

6. Implantat nach einem der Ansprüche 1 bis 5, **dadurch gekennzeichnet, dass** die Retentions-Struktur (9) in Form von Sägezähnen (16) ausgebildet ist.

7. Implantat nach Anspruch 6, **dadurch gekennzeichnet, dass** sich die einzelnen Zähne der Retentions-Struktur (9) über den gesamten Umfang der Fortsätze (4) erstrecken.

8. Implantat nach Anspruch 6 oder 7, **dadurch gekennzeichnet, dass** die über den Umfang der Fortsätze (4) sich erstreckende, sägezahnförmige Retentions-Struktur (9) mindestens an einer Stelle einen Unterbruch aufweist.

9. Implantat nach einem der Ansprüche 6 bis 8, **dadurch gekennzeichnet, dass** die gegen den Grundkörper (1) gerichtete Fläche (14) der Sägezähne (16) einen Winkel α zur Ebene des Grundkörpers (1) einschliesst der kleiner oder gleich gross ist wie der Winkel +β, den die gegen das freie Ende (6) gerichtete Fläche (15) der Sägezähne (16) mit der Ebene des Grundkörpers (1) einschliesst.

10. Implantat nach einem der Ansprüche 6 bis 9, **dadurch gekennzeichnet, dass** der Winkel α, den die gegen den Grundkörper (1) gerichtete Fläche (14) der Sägezähne (16) mit der Ebene des Grundkörpers (1) einschliesst, O° beträgt .

11. Implantat nach einem der Ansprüche 6 bis 9, **dadurch gekennzeichnet, dass** der Winkel α, den die gegen den Grundkörper (1) gerichtete Fläche (14) der Sägezähne (16) mit der Ebene des Grundkörpers (1) einschliesst, einen positiven Wert aufweist.

12. Implantat nach einem der Ansprüche 6 bis 9, **dadurch gekennzeichnet, dass** der Winkel α, den die gegen den Grundkörper (1) gerichtete Fläche (14) der Sägezähne (16) mit der Ebene des Grundkörpers (1) einschliesst, einen negativen Wert aufweist.

13. Implantat nach einem der Ansprüche 1 bis 12, **dadurch gekennzeichnet, dass** die Retentions-Struktur (9) aus gegen die Unterseite (3) gerichteten Widerhaken besteht.

14. Implantat nach einem der Ansprüche 1 bis 13, **dadurch gekennzeichnet, dass** die Retentions-Struktur (9) aus einer Anzahl von sich zur Unterseite (3) hin konisch verbreiternden Kegelstumpfabschnitten besteht.

15. Implantat nach einem der Ansprüche 1 bis 14, **dadurch gekennzeichnet, dass** die Retentions-Struktur (9) aus einer Anzahl von annähernd parallel zur Unterseite (3) verlaufenden Retentionswülsten besteht.

16. Implantat nach einem der Ansprüche 1 bis 15, **dadurch gekennzeichnet, dass** der plattenförmige Grundkörper (1) als Gitter ausgebildet ist.

17. Implantat nach einem der Ansprüche 1 bis 16, **dadurch gekennzeichnet, dass** der Grundkörper (1) eine annähernd rechteckförmige Gestalt besitzt und vorzugsweise zwei Fortsätze (4) vorgesehen sind.

18. Implantat nach einem der Ansprüche 1 bis 16, **dadurch gekennzeichnet, dass** der Grundkörper (1) als Winkelplatte ausgebildet ist und vorzugsweise drei Fortsätze (4) vorgesehen sind.

19. Implantat nach einem der Ansprüche 1 bis 16, **dadurch gekennzeichnet, dass** der Grundkörper (1) sternförmig ausgebildet ist.

20. Implantat nach Anspruch 19, **dadurch gekennzeichnet, dass** an jedem der freien Sternzacken des Grundkörpers (1) ein Fortsatz (4) vorgesehen ist.

21. Implantat nach einem der Ansprüche 1 bis 20, **dadurch gekennzeichnet, dass** die Fortsätze (4) als Hohlkörper mit einem zentralen Kanal (10) ausgebildet sind.

22. Implantat nach Anspruch 21, **dadurch gekennzeichnet, dass** der zentrale Kanal (10) nach oben offen ist.

23. Implantat nach Anspruch 21 oder 22, **dadurch gekennzeichnet, dass** der zentrale Kanal (10) nach unten geschlossen ist.

24. Implantat nach Anspruch 21 oder 22, **dadurch gekennzeichnet, dass** der zentrale Kanal (10) nach unten offen ist.

25. Implantat nach einem der Ansprüche 21 bis 24, **dadurch gekennzeichnet, dass** der zentrale Kanal (10) über seine gesamte Länge den gleichen Querschnitt aufweist.

26. Implantat nach einem der Ansprüche 21 bis 24, **dadurch gekennzeichnet, dass** der zentrale Kanal (10) einen variablen Querschnitt aufweist.

27. Implantat nach einem der Ansprüche 21 bis 24, **dadurch gekennzeichnet, dass** sich der zentrale Kanal (10) nach unten verjüngt.

28. Implantat nach einem der Ansprüche 21 bis 24, **dadurch gekennzeichnet, dass** sich der zentrale Kanal (10) nach oben verjüngt.

29. Implantat nach einem der Ansprüche 21 bis 28, **dadurch gekennzeichnet, dass** der zentrale Kanal (10), vorzugsweise nur in seinem oberen Teil, ein Innengewinde (18) aufweist.

30. Implantat nach einem der Ansprüche 21 bis 29, **dadurch gekennzeichnet, dass** der zentrale Kanal (10) mit einem Spreizkörper (13) verschliessbar ist.

31. Implantat nach Anspruch 30, **dadurch gekennzeichnet, dass** mehrere zentrale Kanäle (10) mit einem Verschlussteil (31) gleichzeitig verschliessbar sind, welcher mehrere untereinander verbundene Spreizkörper (13) aufweist.

32. Implantat nach einem der Ansprüche 21 bis 31, **dadurch gekennzeichnet, dass** es zusätzlich einen Spreizkörper (13) umfasst, der vorzugsweise aus einem röntgenkontrastreichen Material besteht und in den zentralen Kanal (10) einführbar ist.

33. Implantat nach Anspruch 32, **dadurch gekennzeichnet, dass** der Spreizkörper (13) aus einem bioresorbierbaren Material besteht.

34. Implantat nach Anspruch 32 oder 33, **dadurch gekennzeichnet, dass** der Spreizkörper (13) ein Aussengewinde (19) aufweist.

35. Implantat nach einem der Ansprüche 32 bis 34, **dadurch gekennzeichnet, dass** der Spreizkörper (13) an seinem oberen Teil ein Vertiefung (20), vorzugsweise in Form eines Innensechskantes (30) aufweist.

36. Implantat nach einem der Ansprüche 31 bis 35, **dadurch gekennzeichnet, dass** es zusätzlich einen Verschlussteil (31) mit mehreren untereinander verbundenen Spreizkörpern (13) umfasst.

37. Implantat nach einem der Ansprüche 1 bis 36, **dadurch gekennzeichnet, dass** die Fortsätze (3) einen Längsschlitz (17) aufweisen.

38. Implantat nach Anspruch 37, **dadurch gekennzeichnet, dass** die Längsschlitze (17) nach unten offen sind.

39. Implantat nach Anspruch 37, **dadurch gekennzeichnet, dass** die Längsschlitze (17) nach unten geschlossen sind.

40. Implantat nach einem der Ansprüche 1 bis 39, **dadurch gekennzeichnet, dass** es mindestens drei Fortsätze (4), vorzugsweise mindestens vier Fortsätze (4) aufweist.

## Claims

1. An implant for osteosynthesis having a plate-like main body (1) which comprises a top surface (2) and a bottom surface (3) designed for bone contact,
A) the bottom surface (3) of the implant being provided with at least two projections (4) which are designed for implantation into the bone;
B) the outside surface (5) of the projections (4) being provided with a retention structure (9); and
C) the entire implant consisting of a bioresorbable material, **characterised in that**
D) the thickness of the plate-like main body (1) decreases continually from the centre (7) to the periphery (8).

2. The implant as claimed in claim 1, **characterised in that** the projections (4) are essentially arranged perpendicularly to the main body (1).

3. The implant as claimed in claim 1, **characterised in that** the projections (4) converge towards each other, preferably forming an angle (12) of 20 degrees, at the most, relative to a straight line (11) extending perpendicularly to the main body (1).

4. The implant as claimed in claim 3, **characterised in that** the projections (4) form an angle (12) of between 1 and 15 degrees, preferably between 2 and 20 degrees, relative to a straight line (11) extending perpendicularly to the main body (1).

5. The implant as claimed in any of the claims 1 to 4, **characterised in that** the projections (4) are realised in an essentially circular cylindrical form and are preferably provided with acute tips on their free end portions (6).

6. The implant as claimed in any of the claims 1 to 5, **characterised in that** the retention structure (9) is realised in the form of buttresses (16).

7. The implant as claimed in claim 6, **characterised in that** the individual buttresses of the retention structure (9) extend over the entire circumference of the projections (4).

8. The implant as claimed in claim 6 or 7, **characterised in that** the buttress-like retention structure (9) extending over the circumference of the projections (4) is interrupted at least at one location.

9. The implant as claimed in any of the claims 6 to 8, **characterised in that** the surface (14) of the buttresses (16) which is directed towards the main body (1) and the plane of the main body (1) form an angle α which is smaller than, or equal to, the angle +β formed by the surface (15) of the buttresses (16) which is directed towards the free end portion (6) and the plane of the main body (1).

10. The implant as claimed in any of the claims 6 to 9, **characterised in that** the angle α formed by the surface (14) of the buttresses (16) which is directed towards the main body (1) and the plane of the main body (1) equals 0 degrees.

11. The implant as claimed in any of the claims 6 to 9, **characterised in that** the angle α formed by the surface (14) of the buttresses (16) which is directed towards the main body (1) and the plane of the main body (1) has a positive value.

12. The implant as claimed in any of the claims 6 to 9, **characterised in that** the angle α formed by the surface (14) of the buttresses (16) which is directed towards the main body (1) and the plane of the main body (1) has a negative value.

13. The implant as claimed in any of the claims 1 to 12, **characterised in that** the retention structure (9) is realised in the form of barbs directed towards the bottom surface (3).

14. The implant as claimed in any of the claims 1 to 13, **characterised in that** the retention structure (9) is realised in the form of a number of frusto-conical segments widening out towards the bottom surface (3).

15. The implant as claimed in any of the claims 1 to 14, **characterised in that** the retention structure (9) is realised in the form of a number of retention bulges extending approximately parallel to the bottom surface (3).

16. The implant as claimed in any of the claims 1 to 15, **characterised in that** the plate-like main body (1) is realised in the form of a grid.

17. The implant as claimed in any of the claims 1 to 16, **characterised in that** the main body (1) is of approximately rectangular shape and is preferably provided with two projections (4).

18. The implant as claimed in any of the claims 1 to 16, **characterised in that** the main body (1) is realised in the form of an angular nail plate and is preferably provided with three projections (4).

19. The implant as claimed in any of the claims 1 to 15, **characterised in that** the plate-like main body (1) is realised in a star-shaped form.

20. The implant as claimed in claim 19, **characterised in that** a projection (4) is provided on each of the branches of said star.

21. The implant as claimed in any of the claims 1 to 20, **characterised in that** the projections (4) are realised as hollow bodies having a central canal (10).

22. The implant as claimed in claim 21, **characterised in that** the central canal (10) is open toward the top.

23. The implant as claimed in claim 21 or 22, **characterised in that** the central canal (10) is closed toward the bottom.

24. The implant as claimed in claim 21 or 22, **characterised in that** the central canal (10) is open toward the bottom.

25. The implant as claimed in any of the claims 21 to 24, **characterised in that** the central canal (10) has the same cross-section over its entire length.

26. The implant as claimed in any of the claims 21 to 24, **characterised in that** the central canal (10) has a variable cross-section.

27. The implant as claimed in claim 21 or 24, **characterised in that** the central canal (10) is shaped in a form that tapers towards the bottom.

28. The implant as claimed in claim 21 or 24, **characterised in that** the central canal (10) is shaped in a form that tapers towards the top.

29. The implant as claimed in any of the claims 21 to 28, **characterised in that** the central canal (10) is provided with an internal screw thread (18) which preferably extends only over its upper portion.

30. The implant as claimed in any of the claims 21 to 29, **characterised in that** the central canal (10) may be closed by a spreader (13).

31. The implant as claimed in claim 30, **characterised in that** a plurality of central canals (10) may be closed by an end cap element (31) which is provided with several interconnected spreaders (13).

32. The implant as claimed in any of the claims 21 to 31, **characterised in that** it comprises in addition a spreader (13) which preferably consists of a radiopaque material and which may be inserted into the central canal (10).

33. The implant as claimed in claim 32, **characterised in that** the spreader (13) consists of a bioresorbable material.

34. The implant as claimed in claim 32 or 33, **characterised in that** the spreader (13) is provided with an external screw thread (19).

35. The implant as claimed in any of the claims 32 to 34, **characterised in that** the spreader (13) on its top portion is provided with a recess (20) preferably realised in the form of a hexagon socket (30).

36. The implant as claimed in any of the claims 31 to 35, **characterised in that** it comprises in addition an end cap element (31) provided with a plurality of interconnected spreaders (13).

37. The implant as claimed in any of the claims 1 to 36, **characterised in that** the projections (4) have a longitudinal slot (17).

38. The implant as claimed in claim 37, **characterised in that** the central canal (17) is open toward the bottom.

39. The implant as claimed in claim 37, **characterised in that** the central canal (17) is closed toward the bottom.

40. The implant as claimed in any of the claims 1 to 39, **characterised in that** it has at least three projections (4), preferably four projections (4).

## Revendications

1. Implant d'ostéosynthèse, qui présente un corps de base (1) en forme de plaque qui comprend une face supérieure (2) et une face inférieure (3) destinée à être en contact avec l'os, dans lequel :
A) la face inférieure (3) de l'implant présente au moins deux appendices (4) qui sont destinés à l'implantation dans l'os,
B) la surface extérieure (5) des appendices (4) est dotée d'une structure de rétention (9) et
C) l'ensemble de l'implant est constitué d'un matériau biorésorbable, **caractérisé en ce que**
D) l'épaisseur du corps de base (1) en forme de plaque diminue de manière continue du centre (7) vers la périphérie (8).

2. Implant selon la revendication 1, **caractérisé en ce que** les appendices (4) sont disposés essentiellement à la perpendiculaire du corps de base (1).

3. Implant selon la revendication 1, **caractérisé en ce que** les appendices (4) convergent les uns vers les autres et forment par rapport à la perpendiculaire (11) du corps de base (1) un angle (12) de préférence d'au plus 20°.

4. Implant selon la revendication 3, **caractérisé en ce que** les appendices (4) forment par rapport à la perpendiculaire (11) du corps de base (1) un angle (12) de 1° à 15°, de préférence de 2° à 10°.

5. Implant selon l'une des revendications 1 à 4, **caractérisé en ce** les appendices (4) sont de forme essentiellement cylindrique circulaire et se rétrécissent de préférence en pointe à leur extrémité libre (6).

6. Implant selon l'une des revendications 1 à 5, **caractérisé en ce que** la structure de rétention (9) est configurée en dents de scie (16).

7. Implant selon la revendication 6, **caractérisé en ce que** les dents individuelles de la structure de rétention (9) s'étendent sur toute la périphérie des appendices (4).

8. Implant selon la revendication 6 ou 7, **caractérisé en ce que** la structure de rétention (9) en forme de dents de scie qui s'étend à la périphérie des appendices (4) présente une interruption en au moins un endroit.

9. Implant selon l'une des revendications 6 à 8, **caractérisé en ce que** la surface (14) des dents de scie (16) orientée vers le corps de base (1) forme par rapport au plan du corps de base (1) un angle α qui est inférieur ou égal à l'angle β qui est formé entre la surface (15) en dents de scie (16) tournée vers l'extrémité libre (6) et le plan du corps de base (1).

10. Implant selon l'une des revendications 6 à 9, **caractérisé en ce que** l'angle α qui est formé entre la surface (14) des dents de scie (16) qui est tournée vers le corps de base (1) et le plan du corps de base (1) vaut 0°.

11. Implant selon l'une des revendications 6 à 9, **caractérisé en ce que** l'angle α qui est formé entre la surface (14) des dents de scie (16) qui est tournée vers le corps de base (1) et le plan du corps de base (1) présente une valeur positive.

12. Implant selon l'une des revendications 6 à 9, **caractérisé en ce que** l'angle α qui est formé entre la surface (14) des dents de scie (16) qui est tournée vers le corps de base (1) et le plan du corps de base (1) présente une valeur négative.

13. Implant selon l'une des revendications 1 à 12, **caractérisé en ce que** la structure de rétention (9) est constituée de crochets orientés vers le côté inférieur (3).

14. Implant selon l'une des revendications 1 à 13, **caractérisé en ce que** la structure de rétention (9) est constituée d'un certain nombre de troncs de cône qui s'élargissent coniquement en direction du côté inférieur (3).

15. Implant selon l'une des revendications 1 à 14, **caractérisé en ce que** la structure de rétention (9) est constituée d'un certain nombre de bourrelets de rétention qui s'étendent sensiblement en parallèle à la face inférieure (3).

16. Implant selon l'une des revendications 1 à 15, **caractérisé en ce que** le corps de base (1) en forme de plaque est configuré en grille.

17. Implant selon l'une des revendications 1 à 16, **caractérisé en ce que** le corps de base (1) présente une forme sensiblement rectangulaire, deux appendices (4) étant de préférence prévus.

18. Implant selon l'une des revendications 1 à 16, **caractérisé en ce que** le corps de base (1) est configuré comme plaque de cornière, de préférence trois appendices (4) étant prévus.

19. Implant selon l'une des revendications 1 à 16, **caractérisé en ce que** le corps de base (1) est configuré en étoile.

20. Implant selon la revendication 19, **caractérisé en ce qu'**un appendice (4) est prévu à chacune des branches libres de l'étoile du corps de base (1).

21. Implant selon l'une des revendications 1 à 20, **caractérisé en ce que** les appendices (4) sont configurés comme corps creux dotés d'un canal central (10).

22. Implant selon la revendication 21, **caractérisé en ce que** le canal central (10) est ouvert dans le haut.

23. Implant selon la revendication 21 ou 22, **caractérisé en ce que** le canal central (10) est fermé dans le bas.

24. Implant selon la revendication 21 ou 22, **caractérisé en ce que** le canal central (10) est ouvert dans le bas.

25. Implant selon l'une des revendications 21 à 24, **caractérisé en ce que** le canal central (10) présente la même section transversale sur toute sa longueur.

26. Implant selon l'une des revendications 21 à 24, **caractérisé en ce que** le canal central (10) présente une section transversale variable.

27. Implant selon l'une des revendications 21 à 24, **caractérisé en ce que** le canal central (10) se rétrécit vers le bas.

28. Implant selon l'une des revendications 21 à 24, **caractérisé en ce que** le canal central (10) se rétrécit vers le haut.

29. Implant selon l'une des revendications 21 à 28, **caractérisé en ce que** le canal central (10) présente un filet intérieur (18), de préférence uniquement dans sa partie supérieure.

30. Implant selon l'une des revendications 21 à 29, **caractérisé en ce que** le canal central (10) peut être fermé par un corps expansible (13).

31. Implant selon la revendication 30, **caractérisé en ce que** plusieurs canaux centraux (10) peuvent être fermés simultanément par une pièce de fermeture (31) qui présente plusieurs corps expansibles (13) reliés les uns aux autres.

32. Implant selon l'une des revendications 21 à 31, **caractérisé en ce qu'**il comprend en outre un corps expansible (13) qui est de préférence constitué d'un matériau à fort contraste sous rayons X et qui peut être inséré dans le canal central (10).

33. Implant selon la revendication 32, **caractérisé en ce que** le corps expansible (13) est constitué d'un matériau biorésorbable.

34. Implant selon la revendication 32 ou 33, **caractérisé en ce que** le corps expansible (13) présente un filet extérieur (19).

35. Implant selon l'une des revendications 32 à 34, **caractérisé en ce que** le corps expansible (13) présente dans sa partie supérieure un creux (20) qui présente de préférence la forme d'un creux à six pans (30).

36. Implant selon l'une des revendications 31 à 35, **caractérisé en ce qu'**il comporte en outre une pièce de fermeture (31) dotée de plusieurs corps expansibles (13) reliés les uns aux autres.

37. Implant selon l'une des revendications 1 à 36, **caractérisé en ce que** les appendices (3) présentent une fente longitudinale (17).

38. Implant selon la revendication 37, **caractérisé en ce que** les fentes longitudinales (17) sont ouvertes dans le bas.

39. Implant selon la revendication 37, **caractérisé en ce que** les fentes longitudinales (17) sont fermées dans le bas.

40. Implant selon l'une des revendications 1 à 39, **caractérisé en ce qu'**il présente au moins trois appendices (4) et de préférence au moins quatre appendices (4).
